# EUROPEAN PATENT APPLICATION

(11) **EP 4 661 014 A1**
(43) Date of publication of application: **10.12.2025**
(21) Application number: 24766223.2
(22) Date of filing: 29.01.2024
(51) Int. Cl.: G16C 20/50

(54) **METHOD AND APPARATUS FOR DRUG DESIGN, DEVICE, MEDIUM AND PROGRAM PRODUCT**

(30) Priority: 03.03.2023 CN 202310200273
(71) Applicant: Huawei Cloud Computing Technologies Co., Ltd., Guiyang, Guizhou 550025 (CN)
(72) Inventor: QIAO, Nan, Guiyang, Guizhou 550025 (CN); REN, Feixiao, Guiyang, Guizhou 550025 (CN); LIN, Xinyuan, Guiyang, Guizhou 550025 (CN); XIONG, Zhaoping, Guiyang, Guizhou 550025 (CN); QIU, Jing, Guiyang, Guizhou 550025 (CN)
(74) Representative: Goddar, Heinz J.
(86) International application number: PCT/CN2024/074471
(87) International publication number: WO 2024/183500

(57) **Abstract**

Embodiments of this disclosure provide a method and apparatus for drug design, a device, a medium, and a program product. The method for drug design includes: obtaining protein data representing a three-dimensional structure of a protein and initial molecule data representing an initial molecule to be bound to the three-dimensional structure of the protein. The method further includes: determining first molecular fragment data representing a first molecular fragment in the initial molecule based on the protein data and the initial molecule data. The method further includes: generating target molecule data representing a target molecule based on the first molecular fragment data and the initial molecule data. According to embodiments of this disclosure, for the three-dimensional structure of the protein, a molecular fragment is automatically determined in the initial molecule, and the initial molecule is optimized based on the determined molecular fragment, so that fragment-based artificial intelligence optimization of a drug molecule can be implemented in a targeted manner, thereby reducing time and labor costs of drug discovery.

## Description

This application claims priority to Chinese Patent Application No. 202310200273.X, filed with the China National Intellectual Property Administration on March 3, 2023 and entitled "METHOD AND APPARATUS FOR DRUG DESIGN, DEVICE, MEDIUM, AND PROGRAM PRODUCT", which is incorporated herein by reference in its entirety.

### TECHNICAL FIELD

Embodiments of this disclosure mainly relate to the field of drug design. More specifically, embodiments of this disclosure relate to a method and apparatus for drug design, an electronic device, a computer-readable storage medium, and a computer program product.

### BACKGROUND

A conventional drug research and development process usually includes phases such as drug discovery, pre-clinical research, clinical trials, and market launch, approximately requiring a research and development cycle of over 12 years. Average costs of conventional drug research and development are usually billions of dollars, and a final failure rate is greater than 90%. The drug discovery phase includes a target determining process, a compound library construction process, a lead compound discovery process, and a molecular structure optimization process. The molecular structure optimization process includes the following steps: synthesizing a large quantity of new compounds by using a lead compound as a template; studying physical and chemical properties, metabolic properties and early toxicological data of the compounds; and selecting an optimal compound that meets druggability. Therefore, the conventional drug research and development process has high investment, a long period, high risks, and a low success rate.

In addition, during drug design, there are numerous intermolecular permutations and combinations, forming a huge molecular space. High labor costs are needed for study of biological properties of drugs. Therefore, conventional drug discovery relies on knowledge and experience of a pharmaceutical expert to a great extent, with great uncertainty and limitations on novelty of drug discovery.

### SUMMARY

Embodiments of this disclosure provide a solution for drug design.

According to a first aspect of this disclosure, a method for drug design is provided. The method includes: obtaining protein data representing a three-dimensional structure of a protein and initial molecule data representing an initial molecule to be bound to the three-dimensional structure of the protein. The method further includes: determining first molecular fragment data representing a first molecular fragment in the initial molecule based on the protein data and the initial molecule data. The method further includes: generating target molecule data representing a target molecule based on the first molecular fragment data and the initial molecule data. In this manner, for the three-dimensional structure of the protein, a molecular fragment is automatically determined in the initial molecule, and the initial molecule is optimized based on the determined molecular fragment, so that fragment-based artificial intelligence optimization of a drug molecule can be implemented in a targeted manner, thereby reducing time and labor costs of drug discovery.

In some embodiments of the first aspect, the first molecular fragment data representing the first molecular fragment in the initial molecule may be determined in the following manner: determining binding-site data representing a plurality of binding sites of the initial molecule in a pocket of the three-dimensional structure of the protein based on the protein data and the initial molecule data; determining a plurality of pieces of molecular fragment data representing a plurality of molecular fragments of the initial molecule at the plurality of binding sites based on the binding-site data; determining remaining fragment data representing a remaining molecular fragment in the initial molecule other than the first molecular fragment by removing the first molecular fragment from the initial molecule; and generating target molecule data representing a target molecule based on the remaining fragment data and the protein data. In this manner, a to-be-optimized molecular fragment may be efficiently determined from the plurality of molecular fragments by using the binding-site data, to reduce or even eliminate dependence on expert experience.

In some embodiments of the first aspect, the protein data representing the three-dimensional structure of the protein and the initial molecule data representing the initial molecule to be bound to the three-dimensional structure of the protein may be obtained in the following manner, including: receiving a first input for the protein and the initial molecule; and obtaining the protein data and the initial molecule data from a database based on the first input. In this way, the protein data and the initial molecule data can be obtained quickly and efficiently.

In some embodiments of the first aspect, the first molecular fragment data representing the first molecular fragment in the initial molecule may be determined in the following manner, including: determining first candidate molecular fragment data representing at least one candidate molecular fragment in the initial molecule based on the protein data and the initial molecule data; outputting the first candidate molecular fragment data for graphical display of the at least one candidate molecular fragment; and determining one candidate molecular fragment in data of the at least one candidate molecular fragment as the first molecular fragment data based on a second user input for the at least one candidate molecular fragment. In this manner, a to-be-optimized molecular fragment can be more accurately determined by combining artificial intelligence and expert experience.

In some embodiments of the first aspect, the method further includes: receiving a manipulation input for graphical manipulation of at least one of the three-dimensional structure of the protein and the at least one candidate molecular fragment; performing manipulation processing on the at least one to generate a manipulation result; and outputting the manipulation result for graphical display of the manipulated at least one. In this manner, a drug molecule design process becomes intuitive and operable through the graphical manipulation input of a user and graphical display.

In some embodiments of the first aspect, the target molecule data representing the target molecule may be generated in the following manner, including: receiving a substitute molecular fragment input representing at least one substitute molecular fragment that is used for substituting the first molecular fragment and that is to be bound to a remaining molecule; generating candidate target molecule data representing at least one candidate target molecule based on the at least one substitute molecular fragment and the remaining molecular fragment; outputting the candidate target molecule data for graphical representation of the candidate target molecule; and receiving a third user input for the candidate target molecule data, and determining one of the at least one candidate target molecule as the target molecule. In this way, a drug molecule that meets an actual requirement may be better implemented.

In some embodiments of the first aspect, the target molecule data representing the target molecule may be generated in the following manner, including: selecting substitute fragment data representing at least one substitute molecular fragment from the database based on the remaining fragment data and the protein data; outputting the substitute molecular fragment data for graphical display of the at least one substitute molecular fragment; receiving a target selection input for selecting a target substitute molecular fragment or the target molecule; and generating the target molecule data based on the target selection input or based on the target substitute molecular fragment and the remaining molecular fragment. In this way, a drug molecule that meets an actual requirement may be implemented much better.

In some embodiments of the first aspect, the binding-site data includes binding status data representing a status of binding between the protein and the initial molecule at a corresponding binding site in the plurality of binding sites; the binding status data includes binding free energy of the protein and the initial molecule at the corresponding binding site; and the first molecular fragment data may be determined in the following manner: determining the first molecular fragment data from the plurality of pieces of molecular fragment data by comparing the binding free energy at the corresponding binding site with a first threshold. In this manner, a to-be-optimized molecular fragment may be determined efficiently by comparing the binding free energy at the corresponding binding site with the specified threshold, to reduce time and costs.

In some embodiments of the first aspect, the binding status data further includes at least one of the following: at the corresponding binding site, a degree of shape matching between the initial molecule and the pocket; or a spatial margin between a corresponding molecular fragment in the plurality of molecular fragments and the pocket; or polarity data representing a polarity of a corresponding molecular fragment. In this manner, more other types of binding status data may be selected for determining whether one or more molecular fragments in the plurality of molecular fragments are molecular fragments that need to be optimized.

In some embodiments of the first aspect, the first molecular fragment data may be determined in the following manner. The first molecular fragment data is determined from the plurality of pieces of molecular fragment data based on at least one of the following: the degree of shape matching is less than a second threshold; or the spatial margin is less than a third threshold; or the polarity data is less than a fourth threshold. In this manner, accuracy of determining a to-be-optimized molecular fragment may be improved by selecting one or more threshold conditions.

In some embodiments of the first aspect, the target molecule data representing the target molecule may be generated in the following manner: removing the first molecular fragment from the initial molecule, to generate remaining fragment data representing a remaining molecular fragment in the initial molecule other than the first molecular fragment; generating second molecular fragment data representing a second molecular fragment based on the remaining fragment data and context data that is in the protein data and that is associated with the first molecular fragment data; and generating the target molecule data based on the second molecular fragment data and the remaining fragment data. In this manner, the determined molecular fragment is removed from the initial molecule, and the target molecule is generated by using a pre-trained model, so that dependence on a molecular library and a fragment library may be reduced, and an optimized drug molecule is generated simply and efficiently, to reduce time and labor costs of drug design.

In some embodiments of the first aspect, the second molecular fragment data representing the second molecular fragment may be generated in the following manner: determining whether the first molecular fragment data is end data representing an end in the three-dimensional structure of the protein; if the first molecular fragment data is the end data, determining, from the remaining fragment data, first molecular fragment generation information corresponding to the end data; and generating the second molecular fragment data based on the first molecular fragment generation information and the context data. In this manner, for a case in which a to-be-optimized molecular fragment is located at an end of the three-dimensional structure of the protein, a molecular fragment may be regenerated at the end by using at least partial information of the remaining molecular fragment and protein context information that is associated with the removed molecular fragment, to implement fragment optimization of a drug molecule.

In some embodiments of the first aspect, the second molecular fragment data representing the second molecular fragment may be generated in the following manner: determining whether the first molecular fragment data is intermediate data representing an intermediate portion of the three-dimensional structure of the protein; if it is determined that the first molecular fragment data is the intermediate data, determining, from the remaining molecular fragment data, second molecular fragment generation information and third molecular fragment generation information that correspond to the intermediate data; and generating the second molecular fragment data based on the second molecular fragment generation information, the third molecular fragment generation information, and the context data. In this manner, for a case in which a to-be-optimized molecular fragment is located in the intermediate portion of the three-dimensional structure of the protein, by using at least partial information of the remaining molecular fragment and protein context information that is associated with the removed molecular fragment, a molecular fragment may be regenerated between two ends that are exposed after the removal of the molecular fragment from the three-dimensional structure of the protein is complete, to implement fragment optimization of a drug molecule.

In some embodiments of the first aspect, the target molecule data may be generated in the following manner: generating candidate molecule data representing a candidate molecule by adjusting the second molecular fragment data and the remaining fragment data; and determining the target molecule data from the candidate molecule data based on an attribute of the target molecule. In this way, the generated target molecule has higher stability and meets a requirement for the target molecule in practice, to further improve a drug design process.

In some embodiments of the first aspect, the method further includes: generating three-dimensional graphic display of the target molecule and the three-dimensional structure of the protein. In this way, intuitive experience of drug design may be implemented. In addition, all the foregoing methods provided according to the first aspect may be performed in a three-dimensional operating space, thereby improving controllability of the drug design process.

According to a second aspect of this disclosure, an apparatus for drug design is provided. The apparatus includes: a data obtaining unit, configured to obtain protein data representing a three-dimensional structure of a protein and initial molecule data representing an initial molecule to be bound to the three-dimensional structure of the protein; a molecular fragment determining unit, configured to determine first molecular fragment data representing a first molecular fragment in the initial molecule based on the protein data and the initial molecule data; a remaining-fragment determining unit, configured to determine remaining fragment data representing a remaining molecular fragment in the initial molecule other than the first molecular fragment by removing the first molecular fragment from the initial molecule; and a target-molecule generation unit, configured to generate target molecule data representing a target molecule based on the remaining fragment data and the protein data.

According to a third aspect of this application, a computing device cluster is further provided, including at least one computing device, where each computing device includes a processor and a memory. The processor of the at least one computing device is configured to execute instructions stored in the memory of the at least one computing device, to enable the computing device cluster to perform the method according to the first aspect of this application.

According to a fourth aspect of this application, a computer-readable storage medium is further provided, where a computer program is stored on the computer-readable storage medium; and when the program is executed by a processor, the method according to the first aspect of this application is implemented.

According to a fifth aspect of this application, a computer program product is further provided, including computer-executable instructions; and when the computer executable instructions are executed by a processor, the method according to the first aspect of this application is implemented.

It can be understood that, the apparatus according to the second aspect, the computing device cluster according to the third aspect, the computer storage medium according to the fourth aspect, or the computer program product according to the fifth aspect is configured to perform the method according to the first aspect. Therefore, explanations or descriptions of the first aspect are also applicable to the second aspect, the third aspect, the fourth aspect, and the fifth aspect. In addition, for beneficial effects that can be achieved in the second aspect, the third aspect, the fourth aspect, and the fifth aspect, reference may be made to the beneficial effects of the corresponding method. Details are not described herein again.

### BRIEF DESCRIPTION OF DRAWINGS

With reference to the accompanying drawings and the following detailed descriptions, the foregoing and other features, advantages, and aspects of embodiments of this disclosure become more apparent. In the accompanying drawings, same or similar reference numerals indicate same or similar elements.
FIG. 1 shows an example environment in which a plurality of embodiments of this disclosure can be implemented;
FIG. 2A shows a diagram of a drug design process according to an embodiment of this disclosure;
FIG. 2B shows a diagram of an example of a protein pocket according to an embodiment of this disclosure;
FIG. 3 shows a schematic flowchart of a process for identifying a molecular fragment according to an embodiment of this disclosure;
FIG. 4 shows a schematic flowchart of a method for drug design according to an embodiment of this disclosure;
FIG. 5 shows a diagram of an example of fragment optimization based on molecular editing according to an embodiment of this disclosure;
FIG. 6 shows a diagram of another example of fragment optimization based on molecular editing according to an embodiment of this disclosure;
FIG. 7 shows a block diagram of an apparatus for drug design according to an embodiment of this disclosure;
FIG. 8 shows an example block diagram of a computing device cluster that may be used to implement an embodiment of this disclosure; and
FIG. 9 is a block diagram of implementation of network connection of one or more computing devices in the example computing device cluster shown in FIG. 8.

### DESCRIPTION OF EMBODIMENTS

The following describes embodiments of this disclosure in more detail with reference to the accompanying drawings. Although some embodiments of this disclosure are shown in the accompanying drawings, it should be understood that this disclosure can be implemented in various forms, and should not be construed as being limited to embodiments described herein, and instead, these embodiments are provided for a more thorough and complete understanding of this disclosure. It should be understood that the accompanying drawings and embodiments of this disclosure are merely used as examples and are not intended to limit the protection scope of this disclosure.

In the descriptions of embodiments of this disclosure, the term "including" and similar terms thereof shall be understood as non-exclusive inclusions, that is, "including but not limited to". The term "based on" should be understood as "at least partially based on". The term "one embodiment" or "this embodiment" should be understood as "at least one embodiment". The terms "first", "second", and the like may indicate different objects or a same object. The following description may further include other explicit and implied definitions. It should be noted that numbers or values used in this specification are examples, and are not intended to limit the protection scope of this disclosure.

"Machine learning" means processing involving high-performance computing, machine learning, and artificial intelligence algorithms. In this specification, the term "machine learning model" may also be referred to as a "learning model", a "learning network", a "network model", or a "model". A "neural network" or "neural network model" is a deep learning model. Generally, the machine learning model may include a plurality of processing layers, and there are a plurality of processing units at each processing layer. The processing unit is sometimes referred to as a convolutional kernel. In a convolutional layer of a convolutional neural network (convolutional neural network, CNN), a processing unit is referred to as a convolutional kernel or a convolutional filter. A processing unit at each processing layer performs a corresponding change on an input of the processing layer based on a corresponding parameter. An output of the processing layer is provided as an input of a next processing layer. An input of the first processing layer of the machine learning model is a model input of the machine learning model, and an output of the last processing layer is a model output of the machine learning model. An input of an intermediate processing layer is sometimes referred to as a feature extracted by the machine learning model. Values of all parameters of processing units of the machine learning model form a parameter value set of the machine learning model.

Machine learning may be mainly divided into three phases: a training phase, a test phase, and an application phase (also referred to as an inference phase). In the training phase, a given machine learning model may be trained by using a large quantity of training samples, and iteration keeps going on until the machine learning model can obtain, from the training samples, consistent inference similar to inference that can be made by human wisdom. The machine learning model may be considered as being capable of learning, from training data, a mapping or association relationship between an input and an output through training. After training, the parameter value set of the machine learning model is determined. In the test phase, a trained machine learning model may be tested by using a test sample, to determine performance of the machine learning model. In the application phase, the machine learning model may be used to process actual input data based on the parameter value set obtained through training, to provide a corresponding output.

To make this disclosure clearer and more comprehensive, the following terms are described.

Computer-aided drug design (computer aided drug design, CADD): Based on computational chemistry or computational biology, CADD uses capabilities of a computer, such as computing, simulation, and prediction, to assist in and accelerate drug discovery.

Structure-based drug design (structure-based drug design, SBDD): a drug design based on a receptor (usually a protein). Based on a structure and properties of the receptor, a ligand molecule that can be specifically bound to the receptor is found in a massive library of small molecular compounds.

Fragment-based drug discovery (fragment-based drug discovery, FBDD): Starting from a structure and properties of a receptor, a set of molecular fragments that can be specifically bound to the receptor is first found in a molecular fragment library. Then operations such as fragment growth, binding, and connection are performed based on a candidate molecular fragment. Finally, a new drug molecule more strongly bound to the receptor is produced.

Artificial intelligence (artificial intelligence, AI)-driven drug design (AI-driven drug design, AIDD): In AI-assisted drug research and development, a series of AI technologies, such as machine learning, deep learning, image recognition, and cognitive computing, are organically embedded into prediction and the like for each phase of research and development of a new drug, to shorten a research and development process of the new drug and maximize research and development efficiency of the new drug. These phases include, for example, target protein discovery, lead compound determining, lead compound structure optimization, and ADMET, where A represents drug absorption, D represents distribution, M represents metabolism, E represents excretion, and T represents toxicity.

In the research and development process of the new drug, appropriate targets (such as genes and proteins) related to disease physiology are first identified, and then a drug or drug-like molecules that can affect these targets are found. After the appropriate targets are identified and validated, a next step is to find an appropriate drug or appropriate drug-like molecules. These molecules can interact with the targets and cause a needed reaction. In embodiments of this disclosure, AI may help, for example, extract useful features, patterns, and structures that exist in a large biomedical dataset. Similar to application of AI in another scenario, in this disclosure, an implementation process of AI in drug research and development may include, for example: obtaining a target training dataset; modeling by using an AI autonomous learning algorithm; training and optimizing a model for a plurality of times; applying a test set to evaluate model performance; and implementing a predetermined goal based on the model, such as molecular screening, prediction, and analysis. Therefore, a prediction capability of artificial intelligence can effectively improve a success rate of drug development.

With significant improvement of capabilities of CADD, deep learning has also achieved great success in designing new drug molecules. For example, in SBDD, there is a great potential to improve specificity and a success rate of computer drug design by considering a structure of a protein pocket. Specifically, sampling may be performed in the protein pocket to generate new drug molecular compounds. These compounds can satisfy a plurality of geometric constraints imposed by the pocket. A conventional sampling algorithm either performs sampling in a graphic space, or considers only 3D coordinates of an atom, ignoring other detailed chemical structures (such as a type of a key and a functional group). To solve this problem, an E(3) equivariant generation network has been developed. The E(3) equivariant generation network utilizes a new graph neural network to capture chemical and geometric constraints in a three-dimensional pocket, and samples a new candidate drug for representation of the captured pocket, thereby achieving better reaction affinity and other drug characteristics, such as drug-likeness and synthetic accessibility.

Currently, a variety of pocket molecule generation based on a deep learning model is proposed, such as pocket molecule generation based on a diffusion model and pocket molecule generation based on an equivariant network and attention (Attention) mechanism. Some schemes have achieved molecule generation from scratch based on a protein target, but a generated molecule is in a two-dimensional form. Other schemes implement a macro-ring linker (linker) generation function. This function can select only a hydrogen H atom as a connection site, and has no interaction process of molecular editing and context information of the protein pocket. Schrodinger is conventional CADD molecular computing simulation software that covers a full scenario of drug discovery, but a fragment design function of the software is to perform traversal and substitution based on an existing fragment library. This may be understood as virtual screening based on a molecular fragment library, without an auxiliary module of AIDD, and is more suitable for an experienced pharmaceutical expert.

In addition, although structure-based drug design and fragment-based drug design have been fully verified in many drug discovery scenarios, SBDD and FBDD consume a huge amount of computing power and depend on construction of a drug molecular library and a fragment library. SBDD and FBDD mainly play a role of an assistant pharmaceutical expert, but do not have capabilities of directly designing a molecule and optimizing a structure. Currently, AIDD is mainly a single-point breakthrough in terms of algorithms, with a low degree of systematization. A conventional product for drug design supports only molecular generation from scratch, and is suitable for early drug discovery and has no interaction in a three-dimensional scenario. In addition, a drug molecule can perform its unique biological function only when it is bound to a specific protein pocket. Therefore, drug molecule design by combining context information of the protein pocket is more suitable for an actual drug design scenario.

This disclosure provides a drug design solution to solve at least some of the foregoing problems and another potential problem. The drug design solution may determine molecular fragment data representing one or more molecular fragments of the initial molecule based on obtained protein data representing a three-dimensional structure of a protein and initial molecule data representing an initial molecule to be bound to the three-dimensional structure of the protein. The drug design solution may further generate target molecule data representing a target molecule based on the molecular fragment data and the initial molecule data. According to an embodiment of this disclosure, a molecular fragment may be automatically determined in the initial molecule, and the initial molecule is optimized based on the determined molecular fragment, so that fragment-based artificial intelligence optimization of a drug molecule can be implemented in a targeted manner, thereby reducing time and labor costs of drug discovery.

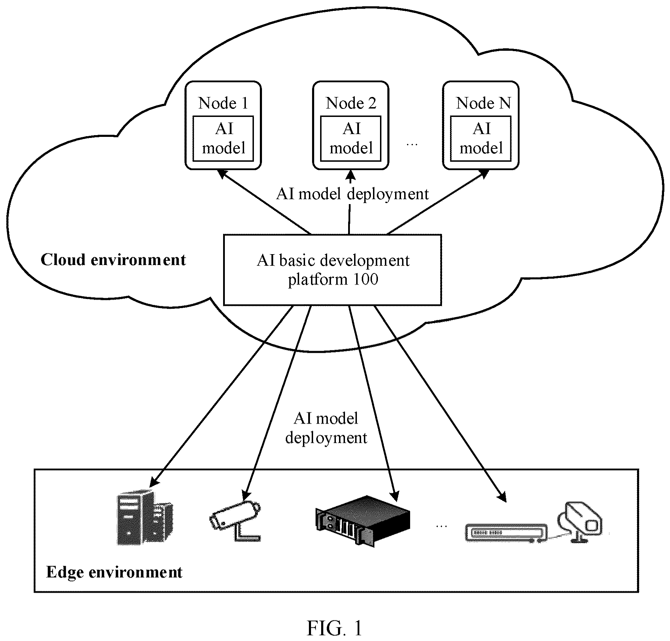

FIG. 1 is a diagram of an example AI platform 100 in which a plurality of embodiments of this disclosure can be implemented. The AI platform 100 shows an example of artificial intelligence optimization for drug design. The example AI platform 100 may be independently deployed on a server or virtual machine in a data center in a cloud environment, or the AI platform 100 may be deployed on a plurality of servers in a data center in a distributed manner, or may be deployed on a plurality of virtual machines in a data center in a distributed manner.

In another embodiment, the AI platform 100 provided in this application may be further deployed in different environments in a distributed manner. The AI platform 100 provided in this application may be logically divided into a plurality of parts, and each part has a different function. For example, a part of the AI platform 100 may be deployed in a computing device (also referred to as an edge computing device) in an edge environment, and the other part may be deployed in a device in the cloud environment. The edge environment is an environment whose geographical location is close to a terminal computing device of a user. The edge environment includes an edge computing device, for example, an edge server, or a small edge station having a computing capability. The parts of the AI platform 100 deployed in different environments or devices collaborate to provide a function such as training an AI model for the user.

Any AI model needs to be trained before it is used to resolve a specific technical problem. AI model training is a process of computing training data by using a specified initial model, and adjusting a parameter in the initial model by using a specific method based on a computing result, so that the model gradually learns a rule and has a specific function. After training, an AI model with a stable function can be used for inference. AI model inference is a process of computing input data by using the trained AI model to obtain a predicted inference result.

In the technical solution of this application, a trained AI model (for example, an AI model deployed on a plurality of nodes (for example, nodes 1, 2, 3, ..., N)) on the AI platform 100 can receive input data (a protein 120 and an initial molecule 130), perform prediction based on the input data, and output a prediction result (a target molecule 140). In this way, intelligent design of a drug molecule in a fragment manner can be implemented by using model training and model management functions provided by the AI platform 100.

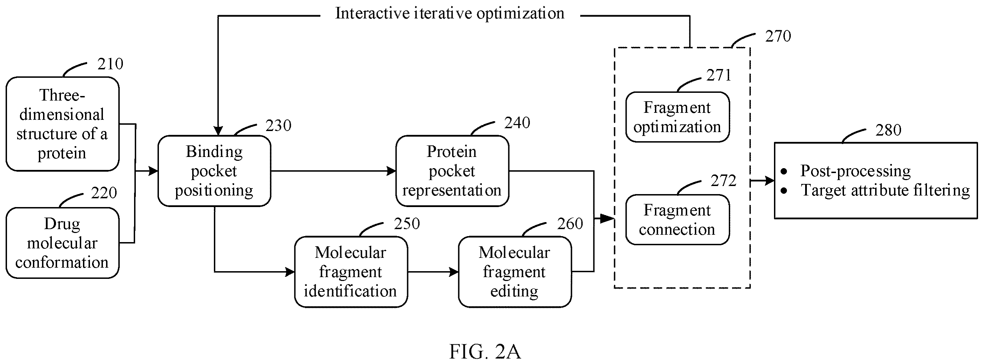

FIG. 2A shows a diagram of a drug design process 200 according to an embodiment of this disclosure. The following describes the process 200 with reference to FIG. 1. The process 200 may be implemented by the example AI platform 100.

As shown in FIG. 2A, the AI platform 100 may obtain user input of a three-dimensional structure 210 of, for example, a protein 120, and a drug molecular conformation 220 (the initial molecule 130). In one example, the three-dimensional structure 210 of the protein and the drug molecular conformation 220 may be obtained by accessing an existing database. In another example, the three-dimensional structure of the protein may be obtained by using a homologous modeling system, or may be obtained (for example, by using a protein structure prediction tool) in a manner of protein structure prediction. The drug molecular conformation can be obtained through a molecular docking system or a molecular generation model (for example, a model for molecule generation from scratch).

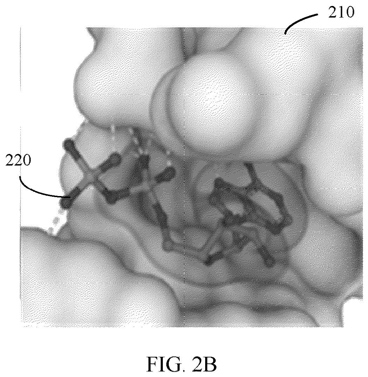

Next, the AI platform 100 may perform binding pocket (pocket) positioning 230 for the three-dimensional structure of the protein and the drug molecular conformation. For example, by using three-dimensional coordinates of a small drug molecule as a center, amino acid residues within a radius of a specific distance from the small drug molecule form the binding pocket. FIG. 2B shows a diagram of an example of a protein pocket according to an embodiment of this disclosure. As shown in FIG. 2B, the protein may be bound to a plurality of molecular fragments of the drug molecule at a plurality of binding sites in the pocket. For example, the protein may be bound to the molecular fragments by using chemical bonding or another bonding manner. In addition, the AI platform 100 may perform representation (240) on the protein pocket to obtain, for example, protein data representing the protein pocket.

Next, the AI platform 100 may perform identification (250) on a modifiable to-be-optimized molecular fragment in which the user is interested. The identified molecular fragment can be used for subsequent processing (for example, molecular fragment editing and molecular fragment generation) in this embodiment. Herein, the user may manually specify the molecular fragment based on the user's own experience. Optionally, the molecular fragment may be selectively specified based on a recommended result that is obtained by the AI platform 100 through comprehensive computing. In another embodiment, the identification (or designation) of the molecular fragment may alternatively be implemented based on both user experience and a computing result of the AI platform 100.

Next, the AI platform 100 may perform editing (260) on the identified molecular fragment. For example, in a three-dimensional scenario, an interactive editing operation, such as an operation of removing an atom, is performed on the identified molecular fragment, to implement atomic-level editing of the drug molecule. Then the following fragment design (270) based on an AI generation model may be implemented based on a size of the identified molecular fragment and a location at which the identified molecular fragment is removed. The fragment design based on the AI generation model includes fragment optimization 271 and fragment connection 272. An optimized candidate molecule may be obtained by using the fragment design based on the AI generation model. In addition, the AI generation model in this disclosure may have access to a plurality of molecular generation models, for example, an autoregressive model and a diffusion model. The AI generation model may learn a chemical space of massive drug molecules in advance, thereby eliminating the dependency of a conventional method on a molecular library and a fragment library.

In one example, if all molecular fragments of the drug molecule in the protein pocket are identified and removed in the block 260, regeneration of all the molecular fragments may be implemented based on the protein data. In another example, if a molecular fragment at an end of the drug molecule in the protein pocket is identified and removed in the block 260, the molecular fragment may be regenerated at the end of the drug molecule based on context information of the protein surrounding the identified molecular fragment in the protein data and information about a remaining molecular fragment of the drug molecule other than the identified molecular fragment, to implement fragment optimization. In still another example, if a molecular fragment in an intermediate portion of the drug molecule in the protein pocket is identified and removed in the block 260, the molecular fragment may be regenerated in the intermediate portion of the drug molecule based on context information of the protein surrounding the identified molecular fragment in the protein data and information about a remaining molecular fragment of the drug molecule other than the identified molecular fragment, to connect the remaining molecular fragment together, thereby implementing fragment optimization and fragment connection. In addition, the AI platform 100 may perform interactive iterative optimization. For example, a molecular optimization result is a three-dimensional conformation, which may be directly connected to an input source for iterative optimization design in practice.

The AI platform 100 may perform post-processing 280, for example, energy minimization processing, on the candidate molecule obtained in the block 270, to optimize a structure of a molecular system, so that the molecular system reaches a balanced and stable state. Alternatively or additionally, the AI platform 100 may perform target attribute filtering 280, to select the target molecule 130 from the post-processed candidate molecule. In an example, an expected value range of a plurality of target-molecule attributes is specified, and a molecule that does not meet the value range is removed from the candidate molecule, to ultimately obtain a target molecule that meets an actual requirement. For example, the expected value or range of the target-molecule attributes may be as follows: molecular weight (molecular weight) [100, 800], molecular druggability (QED) [0.5, 1.0], allocation coefficient (logP) [0, 3], and the like. It can be understood that the foregoing data is merely an example, and is not intended to limit the scope of this disclosure.

According to embodiments of this disclosure, an efficient general-purpose molecular design system that is more suitable for a real drug design scenario may be constructed based on the protein pocket and the molecular fragments. In the general-purpose molecular design system, binding pocket positioning, molecular fragment identification, fragment editing, and AI-based fragment design are concatenated, to implement fragment-based interactive molecular design, which can cover a plurality of design scenarios such as molecular generation from scratch, fragment generation, and fragment connection. The AI generation model at a bottom layer of the system learns a chemical space of massive drug molecules in advance, thereby eliminating the dependency of a conventional method on a molecular library and a fragment library. The user may rely on AI capabilities to perform molecular fragment design in an interaction mode of what you see is what you get by inputting only the three-dimensional structure of the protein and the initial molecule.

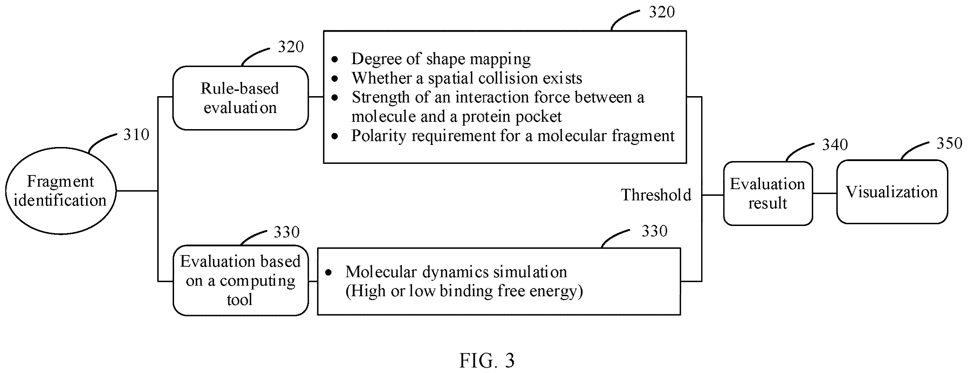

FIG. 3 shows a schematic flowchart of a process 300 for identifying a molecular fragment according to an embodiment of this disclosure. The process 300 may be implemented at the block 250 shown in FIG. 2A. The following describes the process 300 with reference to FIG. 1. The process 300 may be implemented by the example AI platform 100.

As shown in FIG. 3, the process 300 begins with fragment identification 310. Then the AI platform 100 may identify a molecular fragment based on a specific evaluation condition. In some embodiments, the evaluation condition may be the following rule-based evaluation 320: a degree of shape matching between a molecule at a binding site and a protein pocket, whether a spatial collision exists at the binding site, strength of an interaction between the molecule at the binding site and the protein pocket, a polarity requirement for a molecular fragment at the binding site, and the like. In some other embodiments, the evaluation condition may be evaluation 330 based on a computing tool. For example, by using molecular dynamics simulation, the AI platform 100 may compute binding free energy of the molecule and the protein at the binding site. Lower binding free energy indicates more stable binding between the molecule and the protein.

In this specification, "spatial collision" may be referred to as a spatial limitation that can affect normal bonding between the molecule and the protein, and may be expressed by using a spatial margin between the molecule and the protein. Considering that a molecule is a system formed by a nucleus with a positive charge and an electron with a negative charge, an interaction between molecules is generated due to interaction of charges. If the charge distribution of a molecule is balanced and positive and negative charge centers of the molecule are consistent, the molecule is non-polar. On the contrary, the molecule is polar. Interactions exist between molecules with a same polarity, and between molecules with different polarities, and these interactions vary with a distance between the molecules. Therefore, "a polarity requirement for a molecular fragment at the binding site" is used to evaluate whether there is an effective interaction between the molecule and a protein structure surrounding the molecule.

Specifically, the AI platform 100 may obtain an evaluation result 340 based on at least one of the foregoing evaluation conditions. For example, the AI platform 100 determines a result of evaluating a status of binding between the molecule and the protein based on protein data and initial molecule data, for example, the degree of shape matching is 80%. Next, the AI platform 100 may determine whether to identify the molecular fragment by comparing the evaluation result with a predetermined threshold. For example, in a case in which a predetermined threshold for the degree of shape matching is specified as 75%, if a degree of shape matching (for example, 70%) in the evaluation result is less than the predetermined threshold, the molecular fragment is identified in the block 250. On the contrary, if the degree of shape matching (for example, 80%) in the evaluation result is greater than the predetermined threshold, the molecular fragment is not identified in the block 250.

The AI platform 100 may present the identified molecular fragment in a manner of focusing, highlighting, or the like, to facilitate the user's evaluation and selection; and also link up with subsequent fragment editing and fragment design processing. In the foregoing process, a method for automatically identifying a molecular fragment is implemented. The molecular fragment may be identified and located based on a plurality of rules for a molecular binding mode, a computer simulation tool, and the like. On the basis of automatic molecular fragment identification, the user may have selective acceptance based on the user's own pharmaceutical experience or may completely rely on the user's own experience for manual designation. In this way, during molecular fragment identification, a relatively high degree of freedom of interaction is kept between the user and the system.

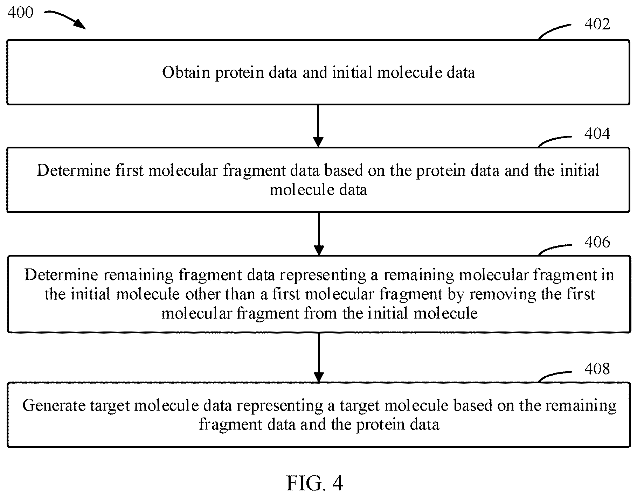

FIG. 4 shows a schematic flowchart of a drug design method 400 according to an embodiment of this disclosure. The drug design method 400 may be implemented by the example AI platform 100 shown in FIG. 1. The following describes the method 400 by using blocks 402 to 406.

At the block 402, protein data representing a three-dimensional structure of a protein and initial molecule data representing an initial molecule to be bound to the three-dimensional structure of the protein are obtained. The three-dimensional structure of the protein at the block 402 may be the three-dimensional structure 210 of the protein in the process 200. The initial molecule at the block 402 may be the initial molecule 220 in the process 200. The protein data and the initial molecule data at the block 402 may be obtained in a manner as described in the process 200. Details are not described herein again. It should be understood that the protein data may further include data representing another attribute of the protein. The initial molecule data may also include other data representing the drug molecule.

In some embodiments, the protein data and the initial molecule data may be obtained in the following manner. First, a first input for the protein and the initial molecule is received. Next, the protein data and the initial molecule data are obtained from a database based on the first input. For example, a user may enter a keyword associated with the protein and the initial molecule, and the AI platform 100 may extract corresponding protein data and initial molecule data from a cloud database based on the keyword entered by the user. In this way, the protein data and the initial molecule data can be obtained quickly and efficiently.

At the block 404, first molecular fragment data representing a first molecular fragment in the initial molecule is determined based on the protein data and the initial molecule data. The first molecular fragment at the block 404 may be the to-be-optimized molecular fragment identified at the block 250 of the process 200.

In some embodiments, the first molecular fragment data representing the first molecular fragment in the initial molecule may be determined in the following manner. First, binding-site data representing a plurality of binding sites of the initial molecule in a pocket of the three-dimensional structure of the protein is determined based on the protein data and the initial molecule data. Next, a plurality of pieces of molecular fragment data representing a plurality of molecular fragments of the initial molecule at the plurality of binding sites are determined based on the binding-site data. The binding-site data includes binding status data representing a status of binding between the protein and the initial molecule at a corresponding binding site in the plurality of binding sites. Here, the binding status data may include data representing the evaluation 320 and the evaluation 330 in the process 300. Next, the first molecular fragment data is determined based on the binding-site data and the plurality of pieces of molecular fragment data. The following uses an example for description.

In some embodiments, the block 404 may include the following processing manners during practical application of a product. First, first candidate molecular fragment data representing at least one candidate molecular fragment in the initial molecule is determined based on the protein data and the initial molecule data. For example, the AI platform 100 may determine whether a molecular fragment 511 is a to-be-optimized candidate molecular fragment by determining the performance of binding between a protein 520 and each molecular fragment of a small molecule 510. If yes, the first candidate molecular fragment data is output for graphical display of the at least one candidate molecular fragment. For example, the AI platform 100 communicates with a display device of a client, to display the candidate molecular fragment 511 on the display device in a manner such as highlighting. The client may display not only a real structure of the candidate molecular fragment 511, but also a specific location of the candidate molecular fragment 511 in the small molecule 510. Next, one candidate molecular fragment in data of the at least one candidate molecular fragment is determined as the first molecular fragment data based on a second user input for the at least one candidate molecular fragment. For example, when the user enters "yes" at the client, the AI platform 100 receives an instruction related to "yes", and determines, based on the instruction, the molecular fragment 511 as a molecular fragment to be removed and reconstructed.

Additionally, during actual application of a product, the AI platform 100 may receive manipulation input for graphical manipulation of at least one of the three-dimensional structure of the protein and the at least one candidate molecular fragment. For example, the AI platform 100 may receive instructions from the client to perform manipulation, such as rotation, movement, and splitting, on the three-dimensional structure of the protein 520 and the candidate molecular fragment 511. Next, the AI platform 100 may perform manipulation processing on the at least one to generate a manipulation result; and further, may output the manipulation result for graphical display of the manipulated at least one. For example, the AI platform 100 implements a moving operation on the candidate molecular fragment 511 based on a received moving instruction, and communicates with the client to display, on the display device, the three-dimensional structure of the protein 520 and the candidate molecular fragment 511 after movement processing.

In one example, the binding status data includes binding free energy of the protein and the initial molecule at the corresponding binding site (refer to the strength of the interaction between the molecule and the protein pocket in the evaluation 320 in the process 300, and the binding free energy in the evaluation 330 in the process 300). The first molecular fragment data may be determined from the plurality of pieces of molecular fragment data by comparing the binding free energy at the corresponding binding site with a first threshold. In another example, the binding status data may further include a degree of shape matching between the initial molecule and the pocket at the corresponding binding site (refer to the degree of shape matching in the evaluation 320 in the process 300). The first molecular fragment data may be determined from the plurality of pieces of molecular fragment data by comparing the degree of shape matching with a second threshold. For example, if the degree of shape matching is less than the second threshold, a corresponding molecular fragment is determined as the first molecular fragment. In still another example, the binding status data may further include a spatial margin between a corresponding molecular fragment in the plurality of molecular fragments and the pocket (refer to the spatial collision in the evaluation 320 in the process 300). The first molecular fragment data may be determined from the plurality of pieces of molecular fragment data by comparing the spatial margin with a third threshold. For example, if the spatial margin is less than the third threshold, the corresponding molecular fragment is determined as the first molecular fragment. In yet another example, the binding status data may further include polarity data representing a polarity of the corresponding molecular fragment (refer to the polarity requirement in the evaluation 320 in the process 300). The first molecular fragment data may be determined from the plurality of pieces of molecular fragment data by comparing the polarity data with a fourth threshold. For example, if the polarity data is less than the fourth threshold, the corresponding molecular fragment is determined as the first molecular fragment.

At the block 406, remaining fragment data representing a remaining molecular fragment in the initial molecule other than the first molecular fragment is determined by removing the first molecular fragment from the initial molecule. The remaining molecular fragment at the block 406 may be the remaining molecular fragment that is obtained after the block 260 of the process 200. The remaining fragment data may be used for generating target molecule data at the block 408.

At the block 408, the target molecule data representing a target molecule is generated based on the remaining fragment data and the protein data. The target molecule at the block 408 may be the target molecule that is obtained after the block 280 of the process 200. In some embodiments, the target molecule data representing the target molecule may be generated in the following manner. First, second molecular fragment data representing a second fragment is generated based on the remaining fragment data and context data that is in the protein data and that is associated with the first molecular fragment data. Next, the target molecule data is generated based on the second molecular fragment data and the remaining fragment data. In this specification, the context data and the remaining fragment data may be amino acid characteristics, coordinate information, atomic properties, atomic coordinates, chemical bonding characteristics, and the like that are respectively related to the protein and the residual molecular fragment at the corresponding binding site. The second fragment at the block 408 may be a fragment that is regenerated in the block 270 of the process 200.

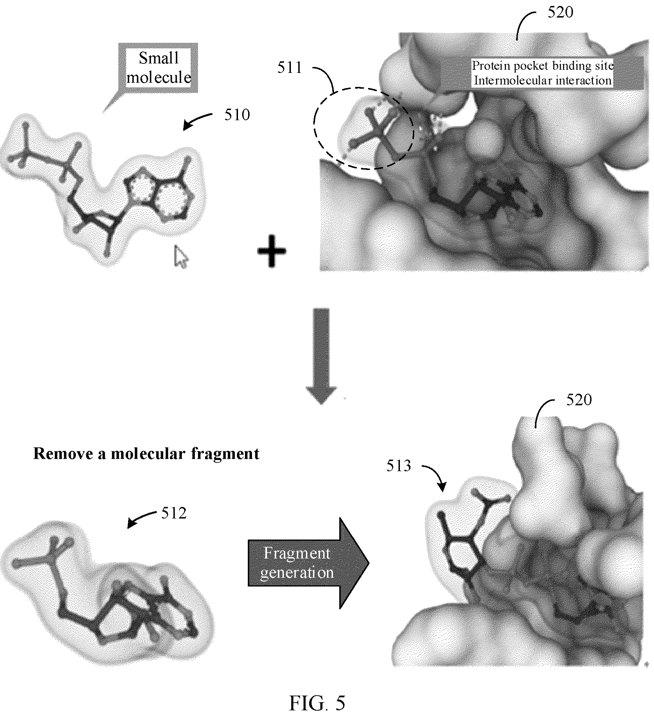

In some embodiments, the second molecular fragment data representing the second molecular fragment may be generated in the following manner. First, it is determined whether the first molecular fragment data is end data representing an end in the three-dimensional structure of the protein. If the first molecular fragment data is the end data, first molecular fragment generation information corresponding to the end data is determined from the remaining fragment data. Next, the second molecular fragment data is generated based on the first molecular fragment generation information and the context data. FIG. 5 shows a diagram of an example of fragment optimization based on molecular editing according to an embodiment of this disclosure. As shown in FIG. 5, at a binding site of a protein pocket, a small drug molecule 510 is bound to a protein 520 through intermolecular interaction. A molecular fragment (refer to 511 in FIG. 5) located at an end in a three-dimensional structure of the protein is determined as a first molecular fragment to be removed. The molecular fragment 511 is removed from the small molecule 510 to generate a remaining molecular fragment 512. Then protein context information at a corresponding position is used, feature information of the remaining molecular fragment 512 is used as first molecular fragment generation information, and a new molecular fragment 513 is regenerated at a position of the molecular fragment 511 through fragment generation. A binding strength of the small molecule at the corresponding position of the protein may be represented through vina scoring. For example, a vina score for the initial small molecule may be -7.15, and a vina score for the optimized small molecule may be -7.51. The lower the vina score, the higher the binding strength.

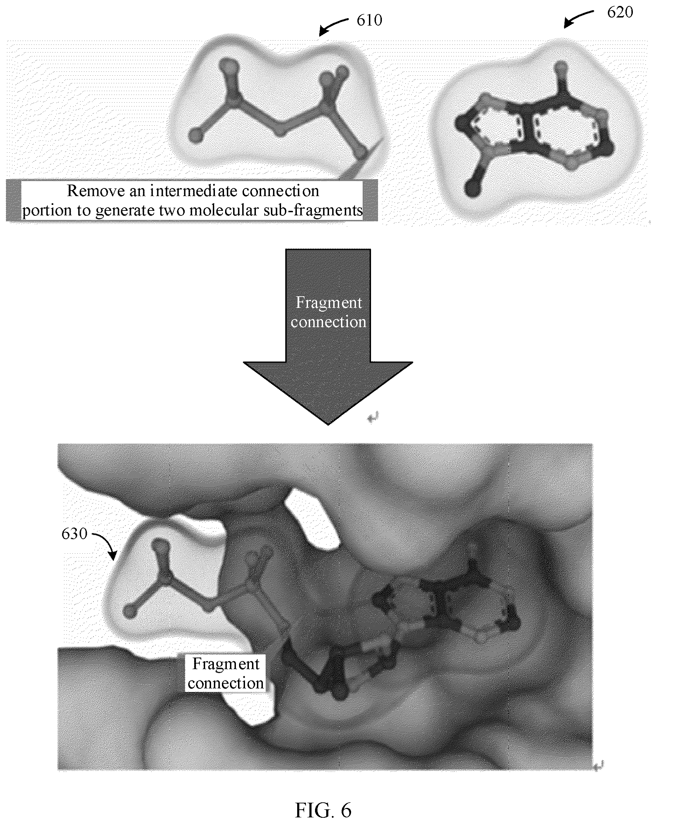

In some other embodiments, the second molecular fragment data representing the second molecular fragment may be generated in the following manner. First, it is determined whether the first molecular fragment data is intermediate data representing an intermediate portion of the three-dimensional structure of the protein. If it is determined that the first molecular fragment data is the intermediate data, second molecular fragment generation information and third molecular fragment generation information that correspond to the intermediate data are determined from the remaining molecular fragment data. Next, the second molecular fragment data is generated based on the second molecular fragment generation information, the third molecular fragment generation information, and the context data. FIG. 6 shows a diagram of another example of fragment optimization based on molecular editing according to an embodiment of this disclosure. As shown in FIG. 6, a molecular fragment located in an intermediate portion of a three-dimensional structure of a protein is determined as a first molecular fragment to be removed. The molecular fragment is removed from a small molecule to generate two remaining molecular fragments (refer to 610 and 620 in FIG. 6). Then protein context information at a corresponding position is used, feature information of the remaining molecular fragments 610 and 620 is used as second molecular fragment generation information and third molecular fragment generation information respectively, and a new molecular fragment 630 is regenerated at a position of the removed molecular fragment through fragment connection.

In some embodiments, the target molecule data may be generated in the following manner. First, candidate molecule data representing a candidate molecule is generated by adjusting the second molecular fragment data and the remaining fragment data. Next, the target molecule data is determined from the candidate molecule data based on an attribute of the target molecule. For details about the foregoing manner, refer to the energy minimization processing and the target attribute filtering 280 in the process 200. Details are not described herein again. In some embodiments, the method 400 may further generate three-dimensional graphic display of the three-dimensional structure of the target molecule and the protein, to implement intuitive experience of drug design and improve controllability of the drug design process.

In an actual application period of a product, the drug molecule design solution in this disclosure may be implemented not only in an artificial intelligence manner, but also in a manual intervention manner. Specifically, there may be the following manners. In some embodiments, first, a substitute molecular fragment input representing at least one substitute molecular fragment that is used for substituting the first molecular fragment and that is to be bound to a remaining molecule is received. Next, candidate target molecule data representing at least one candidate target molecule is generated based on the at least one substitute molecular fragment and the remaining molecular fragment. For example, the AI platform 100 may receive a molecular fragment 513 for substituting a molecular fragment 511, and then generate a new small molecule by splicing the molecular fragment 513 with a molecular fragment 512. Next, the candidate target molecule data is output for graphical representation of the candidate target molecule. For example, the AI platform 100 communicates with the client to display a real new small molecule on the display device. It should be understood that a plurality of new small molecules may be generated, and therefore further screening at the client may be further needed. For example, the user may view, by using the client, a new small molecule that is graphically displayed, and may obtain an attribute (for example, molecular weight) related to the new small molecule. Then the user may issue, based on the related attribute, an instruction to perform screening for an actually needed drug molecule. Correspondingly, the AI platform 100 may receive third user input for the candidate target molecule data, and determine one of the at least one candidate target molecule as the target molecule.

In some other embodiments, first, substitute fragment data representing at least one substitute molecular fragment is selected from the database based on the remaining fragment data and the protein data. For example, the AI platform 100 may select the molecular fragment 513 from the cloud database based on the molecular fragment 512 and the protein 520. Next, the substitute molecular fragment data is output for graphical display of the at least one substitute molecular fragment. For example, the AI platform 100 may communicate with the client, and display a real three-dimensional structure of the molecular fragment 513 and a location of the molecular fragment 513 in the new small molecule on the display device, so that the user determines whether the substitute molecular fragment meets a requirement. If the user enters an instruction related to "yes" on the client, a new small molecule is generated by using the substitute molecular fragment. If the user enters an instruction related to "no" on the client, a new small molecule is generated by using a new substitute molecular fragment to be re-determined. Correspondingly, the AI platform 100 may receive a target selection input for selecting a target substitute molecular fragment, and generate the target molecule data based on the target selection input. Alternatively or additionally, similarly, for screening of a generated new small molecule, the AI platform 100 may communicate with the client, and intuitively display the newly-generated real small molecule on the display device, so that the user determines, based on an attribute, whether the new small molecule meets a requirement. If the user enters an instruction related to "yes" on the client, the new small molecule is determined as the target molecule. If the user enters an instruction related to "No" on the client, re-screening is performed. Correspondingly, the AI platform 100 may receive a target selection input for selecting the target molecule, and generate the target molecule data based on the target selection input.

According to the method in the foregoing embodiment, for the three-dimensional structure of the protein, a molecular fragment is automatically determined in the initial molecule, and the initial molecule is optimized based on the determined molecular fragment, so that fragment-based artificial intelligence optimization of a drug molecule can be implemented in a targeted manner, thereby reducing time and labor costs of drug discovery. The target molecule is generated by using a pre-trained model, thereby reducing dependence on a molecular library and a fragment library and improving accuracy of determining a to-be-optimized molecular fragment. In addition, molecular fragment design is performed in an interaction mode of what you see is what you get through three-dimensional display, and therefore the drug design is more operable. The drug design method according to the embodiment of this disclosure may be performed in a different order from that in FIG. 4, or may be performed in a parallel manner. The drug design method 400 may include more or fewer processes.

The pre-trained AI model invoked in the foregoing processes 200 to 300 may be an AI model developed and deployed on the AI platform 100 in advance, or may be an AI model built in an AI application cloud service.

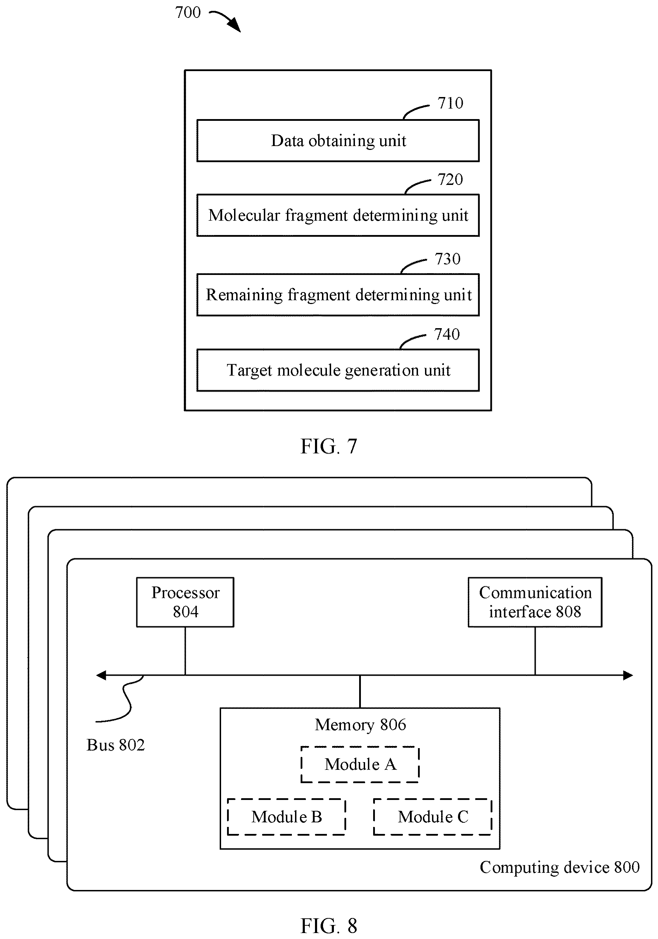

FIG. 7 shows a block diagram of an apparatus 700 for drug design according to an embodiment of this disclosure. The apparatus 700 for drug design may be included in the AI platform 100 or implemented by the AI platform 100.

The apparatus 700 for drug design includes a data obtaining unit 710, a molecular fragment determining unit 720, a remaining-fragment determining unit 730, and a target-molecule generation unit 740. The data obtaining unit 710 is configured to obtain protein data representing a three-dimensional structure of a protein and initial molecule data representing an initial molecule to be bound to the three-dimensional structure of the protein. The molecular fragment determining unit 720 is configured to determine first molecular fragment data representing a first molecular fragment in the initial molecule based on the protein data and the initial molecule data. The remaining-fragment determining unit 730 is configured to determine remaining fragment data representing a remaining molecular fragment in the initial molecule other than the first molecular fragment by removing the first molecular fragment from the initial molecule. The target-molecule generation unit 740 is configured to generate target molecule data representing a target molecule based on the remaining fragment data and the protein data.

In some embodiments, the data obtaining unit 710 may be further configured to obtain the protein data and the initial molecule data from a database. In some embodiments, the data obtaining unit 710 may be further configured to obtain the protein data and the initial molecule data from the database based on a first user input.

In some embodiments, the molecular fragment determining unit 720 may be further configured to determine binding-site data representing a plurality of binding sites of the initial molecule in a pocket of the three-dimensional structure of the protein based on the protein data and the initial molecule data. The molecular fragment determining unit 720 may be further configured to determine a plurality of pieces of molecular fragment data representing a plurality of molecular fragments of the initial molecule at the plurality of binding sites based on the binding-site data. The molecular fragment determining unit 720 may be further configured to determine the first molecular fragment data based on the binding-site data and the plurality of pieces of molecular fragment data.

In some embodiments, the binding-site data includes binding status data representing a status of binding between the protein and the initial molecule at a corresponding binding site in the plurality of binding sites. The binding status data includes binding free energy of the protein and the initial molecule at the corresponding binding site. The molecular fragment determining unit 720 may be further configured to determine the first molecular fragment data from the plurality of pieces of molecular fragment data by comparing the binding free energy at the corresponding binding site with a first threshold.

In some embodiments, the binding status data further includes at least one of the following: at the corresponding binding site, a degree of shape matching between the initial molecule and the pocket; or a spatial margin between a corresponding molecular fragment in the plurality of molecular fragments and the pocket; or polarity data representing a polarity of a corresponding molecular fragment. In some embodiments, the molecular fragment determining unit 720 may be further configured to determine the first molecular fragment data from the plurality of pieces of molecular fragment data based on at least one of the following: the degree of shape matching is less than a second threshold; or the spatial margin is less than a third threshold; or the polarity data is less than a fourth threshold.

In some embodiments, the molecular fragment determining unit 720 may be further configured to output first candidate molecular fragment data representing a first candidate molecular fragment in the initial molecule based on the protein data and the initial molecule data. The molecular fragment determining unit 720 may be further configured to determine the first candidate molecular fragment data as the first molecular fragment data based on a second user input.

In some embodiments, the target-molecule generation unit 740 may be further configured to remove the first molecular fragment from the initial molecule, to generate the remaining fragment data representing the remaining molecular fragment in the initial molecule other than the first molecular fragment. The target-molecule generation unit 740 may be further configured to generate second molecular fragment data representing a second molecular fragment based on the remaining fragment data and context data that is in the protein data and that is associated with the first molecular fragment data. The target-molecule generation unit 740 may be further configured to generate the target molecule data based on the second molecular fragment data and the remaining fragment data.

In some embodiments, the target-molecule generation unit 740 may be further configured to determine whether the first molecular fragment data is end data representing an end in the three-dimensional structure of the protein. The target-molecule generation unit 740 may be further configured to: if the first molecular fragment data is the end data, determine, from the remaining fragment data, first molecular fragment generation information corresponding to the end data. The target-molecule generation unit 740 may be further configured to generate the second molecular fragment data based on the first molecular fragment generation information and the context data.

In some embodiments, the target-molecule generation unit 740 may be further configured to determine whether the first molecular fragment data is intermediate data representing an intermediate portion of the three-dimensional structure of the protein. The target-molecule generation unit 740 may be further configured to: if it is determined that the first molecular fragment data is the intermediate data, determine, from the remaining molecular fragment data, second molecular fragment generation information and third molecular fragment generation information that correspond to the intermediate data. The target-molecule generation unit 740 may be further configured to generate the second molecular fragment data based on the second molecular fragment generation information, the third molecular fragment generation information, and the context data.

In some embodiments, the target-molecule generation unit 740 may be further configured to generate candidate molecule data representing a candidate molecule by adjusting the second molecular fragment data and the remaining fragment data. The target-molecule generation unit 740 may be further configured to determine the target molecule data from the candidate molecule data based on an attribute of the target molecule.

In some embodiments, the target-molecule generation unit 740 may be further configured to output candidate molecule data representing a candidate molecule based on the remaining fragment data and the protein data. The target-molecule generation unit 740 may be further configured to determine the candidate molecule data as the target molecule data based on the second user input.

In some embodiments, the apparatus 700 for drug design may further include a three-dimensional image display unit. The three-dimensional image display unit may be configured to generate three-dimensional graphic display of the target molecule and the three-dimensional structure of the protein.

Because the modules in the AI platform 100 provided in this application may be deployed on a plurality of computing devices in a same environment or different environments in a distributed manner, an embodiment of this application further provides a computing device cluster. The computing device cluster includes one or more computing devices 800 to perform the method performed by the foregoing AI platform. As shown in FIG. 8, the computing device 800 includes a bus 802, a processor 804, a memory 806, and a communication interface 808. The processor 804, the memory 806, and the communication interface 808 implement connections for mutual communication via the bus 802. The computing device may be a server, for example, a central server, an edge server, or a local server in a local data center. In some embodiments, the computing device may alternatively be a terminal device, for example, a desktop computer, a notebook computer, or a smartphone. It should be understood that a quantity of processors and a quantity of memories in the computing device 800 are not limited in this application.

The bus 802 may be a peripheral component interconnect (peripheral component interconnect, PCI) bus, an extended industry standard architecture (extended industry standard architecture, EISA) bus, or the like. Buses may be classified into an address bus, a data bus, a control bus, and the like. For ease of representation, only one line is used for representation in FIG. 8, but this does not indicate that there is only one bus or only one type of bus. The bus 802 may include a path for information transmission between the components (for example, the memory 806, the processor 804, and the communication interface 808) of the computing device 800.

The processor 804 may include any one or more of processors such as a central processing unit (central processing unit, CPU), a graphics processing unit (graphics processing unit, GPU), a microprocessor (microprocessor, MP), or a digital signal processor (digital signal processor, DSP).

The memory 806 may include a volatile memory (volatile memory), such as a random access memory (random access memory, RAM). The processor 804 may further include a non-volatile memory (non-volatile memory), such as a read-only memory (read-only memory, ROM), a flash memory, a hard disk drive (hard disk drive, HDD), or a solid state drive (solid state drive, SSD).

The memory 806 stores executable program code, and the processor 804 executes the executable program code to separately implement functions of the data obtaining unit, the molecular fragment determining unit, the remaining-fragment determining unit, and the target-molecule generation unit, to implement the drug design method. In other words, the memory 806 stores instructions for performing the drug design method. In some possible implementations, the memories 806 in the one or more computing devices 800 in the computing device cluster may separately store a part of the instructions for performing the drug design method. In other words, a combination of the one or more computing devices 800 may jointly execute the instructions for performing the drug design method.

It should be noted that the memories 806 in different computing devices 800 in the computing device cluster may store different instructions respectively used to perform some functions of the drug design apparatus. In other words, the instructions stored in the memories 806 in different computing devices 800 may implement functions of one or more of the data obtaining unit, the molecular fragment determining unit, the remaining-fragment determining unit, and the target-molecule generation unit.

The communication interface 808 uses a transceiver module, for example, but not limited to, a network interface card or a transceiver, to implement communication between the computing device 800 and another device or a communication network.

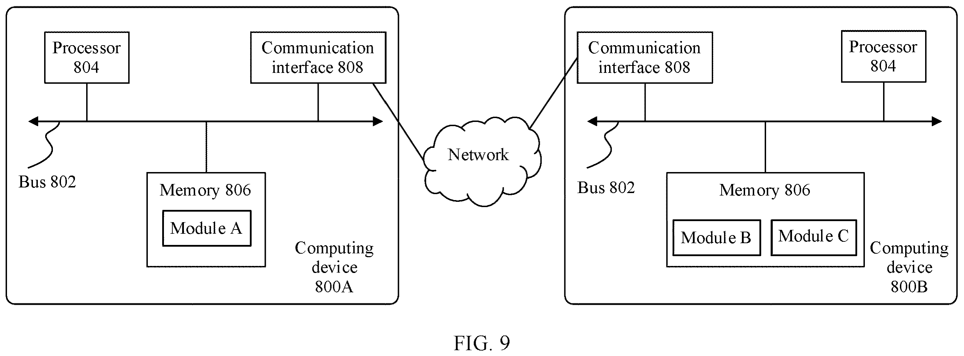

In some possible implementations, the one or more computing devices in the computing device cluster may be connected through a network. The network may be a wide area network, a local area network, or the like. FIG. 9 shows a possible implementation. As shown in FIG. 9, two computing devices 800A and 800B are connected through a network. Specifically, each computing device is connected to the network via a communication interface of the computing device. In such a type of possible implementations, a memory 806 in the computing device 800A stores instructions for performing a function of the data obtaining unit. In addition, a memory 806 in the computing device 800B stores instructions for executing functions of the molecular fragment determining unit, the remaining-fragment determining unit, and the target-molecule fragment generating unit.

It should be understood that, for a manner of connection between the computing device clusters shown in FIG. 9, an actual requirement (for example, whether a large amount of data computing is needed) for the drug design method provided in this application may be considered, to determine whether to assign the functions implemented by the units to the computing device 800A or the computing device 800B for execution. It should be understood that functions of the computing device 800A shown in FIG. 9 may alternatively be completed by a plurality of computing devices 800. Similarly, functions of the computing device 800B may alternatively be completed by a plurality of computing devices 800.

This disclosure may be a method, an apparatus, a system, and/or a computer program product. The computer program product may include a computer-readable storage medium, and the computer-readable storage medium carries computer-readable program instructions for performing various aspects of this disclosure.

The computer-readable storage medium may be a tangible device that can retain and store instructions to be used by an instruction execution device. The computer-readable storage medium may be, for example, but is not limited to, an electronic storage device, a magnetic storage device, an optical storage device, an electromagnetic storage device, a semiconductor storage device, or any appropriate combination thereof. More specific examples (a non-exhaustive list) of the computer-readable storage medium include: a portable computer disk, a hard disk, a random access memory (RAM), a read-only memory (ROM), an erasable programmable read-only memory (EPROM or flash memory), a static random access memory (SRAM), a portable compact disk read-only memory (CD-ROM), a digital versatile disk (DVD), a memory stick, a floppy disk, a mechanical encoded device such as a punched card or groove protrusion structure on which instructions are stored, and any appropriate combination thereof. The computer-readable storage medium used herein is not to be construed as a transient signal, such as a radio wave or another freely propagating electromagnetic wave, an electromagnetic wave propagating through a waveguide or another transmission medium (such as a light pulse through an optical fiber), or an electrical signal transmitted through a wire.

The computer-readable program instructions described herein may be downloaded from the computer-readable storage medium to respective computing/processing devices or to an external computer or external storage device through a network such as the Internet, a local area network, a wide area network, and/or a wireless network. The network may include a copper transmission cable, optical fiber transmission, wireless transmission, a router, a firewall, a switch, a gateway computer and/or an edge server. A network adapter card or network interface in each computing/processing device receives the computer-readable program instructions from the network, and forwards the computer-readable program instructions for storage in a computer-readable storage medium in each computing/processing device.

The computer program instructions used to perform an operation in this disclosure may be assembly instructions, instruction set architecture (ISA) instructions, machine instructions, machine-related instructions, microcode, firmware instructions, status setting data, or source code or target code written in any combination of one or more programming languages. The programming languages include an object-oriented programming language such as Smalltalk and C++, and a conventional procedural programming language such as a "C" language or a similar programming language. The computer-readable program instructions may be executed entirely on a user computer, may be executed partially on a user computer, may be executed as a standalone software package, may be executed partially on a user computer and partially on a remote computer, or may be executed entirely on a remote computer or a server. When a remote computer is involved, the remote computer may be connected to a user computer through any type of network, including a local area network (LAN) or a wide area network (WAN), or may be connected to an external computer (for example, connected by using an Internet service provider through the Internet). In some embodiments, an electronic circuit, for example, a programmable logic circuit, a field programmable gate array (FPGA), or a programmable logic array (PLA), is customized by using status information of the computer-readable program instructions. The electronic circuit may execute the computer-readable program instructions, to implement various aspects of this disclosure.

The aspects of this disclosure are described herein with reference to the flowcharts and/or block diagrams of the method, the apparatus (system), and the computer program product according to embodiments of this disclosure. It should be understood that each block of the flowcharts and/or block diagrams and a combination of blocks of the flowcharts and/or block diagrams may be implemented by using the computer-readable program instructions.

These computer-readable program instructions may be provided to a processing unit of a general-purpose computer, a special-purpose computer, or another programmable data processing apparatus to produce a machine, so that the instructions, when executed by the processing unit of the computer or the another programmable data processing apparatus, create an apparatus for implementing functions/actions specified in one or more blocks in the flowcharts and/or block diagrams. These computer-readable program instructions may alternatively be stored in the computer-readable storage medium. These instructions enable a computer, a programmable data processing apparatus, and/or another device to work in a specific manner. Therefore, the computer-readable medium storing the instructions includes an artifact that includes instructions for implementing the aspects of the functions/actions specified in the one or more blocks in the flowcharts and/or the block diagrams.

Alternatively, the computer-readable program instructions may be loaded onto a computer, another programmable data processing apparatus, or another device, so that a series of operation steps are performed on the computer, the another programmable data processing apparatus, or the another device to produce a computer-implemented process. Therefore, the instructions executed on the computer, the another programmable data processing apparatus, or the another device implement functions/actions specified in one or more blocks of the flowcharts and/or block diagrams.

The flowcharts and block diagrams in the accompanying drawings show possible implementation of system architecture, functions, and operations of the system, method, and computer program product according to a plurality of embodiments of this disclosure. In this regard, each block in the flowcharts or block diagrams may represent a module, a program segment, or a part of the instructions; and the module, the program segment, or the part of the instructions includes one or more executable instructions for implementing a specified logical function. In some alternative implementations, a function marked in a block may occur in a sequence different from that marked in the accompanying drawings. For example, two consecutive blocks may actually be executed substantially in parallel, and sometimes may be executed in a reverse order, depending on a function involved. It should also be noted that, each block in the block diagrams and/or flowcharts, and a combination of the blocks in the block diagrams and/or flowcharts may be implemented by a special-purpose hardware-based system that performs a specified function or action, or may be implemented by a combination of special-purpose hardware and computer instructions.

The foregoing has described the implementations of this disclosure. The foregoing descriptions are examples, not exhaustive, and not limited to the disclosed implementations. Many modifications and variations are apparent to a person of ordinary skill in the art without departing from the scope and spirit of the described implementations. The selection of terms used in this specification is intended to best explain principles of the implementations, practical application or improvements to technologies in the market, or to enable another person of ordinary skill in the art to understand the implementations disclosed in this specification.

## Claims

1. A method for drug design, comprising:
obtaining protein data representing a three-dimensional structure of a protein and initial molecule data representing an initial molecule to be bound to the three-dimensional structure of the protein;
determining first molecular fragment data representing a first molecular fragment in the initial molecule based on the protein data and the initial molecule data;
determining remaining fragment data representing a remaining molecular fragment in the initial molecule other than the first molecular fragment by removing the first molecular fragment from the initial molecule; and
generating target molecule data representing a target molecule based on the remaining fragment data and the protein data.

2. The method according to claim 1, wherein obtaining the protein data representing the three-dimensional structure of the protein and the initial molecule data representing the initial molecule to be bound to the three-dimensional structure of the protein comprises:
receiving a first input for the protein and the initial molecule; and
obtaining the protein data and the initial molecule data from a database based on the first input.

3. The method according to claim 1 or 2, wherein determining the first molecular fragment data representing the first molecular fragment in the initial molecule comprises:
determining first candidate molecular fragment data representing at least one candidate molecular fragment in the initial molecule based on the protein data and the initial molecule data;
outputting the first candidate molecular fragment data for graphical display of the at least one candidate molecular fragment; and
determining one candidate molecular fragment in data of the at least one candidate molecular fragment as the first molecular fragment data based on a second user input for the at least one candidate molecular fragment.

4. The method according to claim 3, further comprising:
receiving a manipulation input for graphical manipulation of at least one of the three-dimensional structure of the protein and the at least one candidate molecular fragment;
performing manipulation processing on the at least one to generate a manipulation result; and
outputting the manipulation result for graphical display of the manipulated at least one.

5. The method according to any one of claims 1 to 4, wherein generating the target molecule data representing the target molecule comprises:
receiving a substitute molecular fragment input representing at least one substitute molecular fragment that is used for substituting the first molecular fragment and that is to be bound to a remaining molecule;
generating candidate target molecule data representing at least one candidate target molecule based on the at least one substitute molecular fragment and the remaining molecular fragment;
outputting the candidate target molecule data for graphical representation of the candidate target molecule; and
receiving a third user input for the candidate target molecule data, and determining one of the at least one candidate target molecule as the target molecule.

6. The method according to any one of claims 1 to 4, wherein generating the target molecule data representing the target molecule comprises:
selecting substitute fragment data representing at least one substitute molecular fragment from the database based on the remaining fragment data and the protein data;
outputting the substitute molecular fragment data for graphical display of the at least one substitute molecular fragment;
receiving a target selection input for selecting a target substitute molecular fragment or the target molecule; and
generating the target molecule data based on the target selection input or based on the target substitute molecular fragment and the remaining molecular fragment.

7. The method according to any one of claims 1 to 6, wherein determining the first molecular fragment data representing the first molecular fragment in the initial molecule comprises:
determining binding-site data representing a plurality of binding sites of the initial molecule in a pocket of the three-dimensional structure of the protein based on the protein data and the initial molecule data;
determining a plurality of pieces of molecular fragment data representing a plurality of molecular fragments of the initial molecule at the plurality of binding sites based on the binding-site data; and
determining the first molecular fragment data based on the binding-site data and the plurality of pieces of molecular fragment data.

8. The method according to claim 7, wherein the binding-site data comprises binding status data representing a status of binding between the protein and the initial molecule at a corresponding binding site in the plurality of binding sites, the binding status data comprises binding free energy of the protein and the initial molecule at the corresponding binding site, and determining the first molecular fragment data comprises:
determining the first molecular fragment data from the plurality of pieces of molecular fragment data by comparing the binding free energy at the corresponding binding site with a first threshold.

9. The method according to claim 8, wherein the binding status data further comprises at least one of the following: at the corresponding binding site,
a degree of shape matching between the initial molecule and the pocket; or
a spatial margin between a corresponding molecular fragment in the plurality of molecular fragments and the pocket; or
polarity data representing a polarity of a corresponding molecular fragment.

10. The method according to claim 9, wherein determining the first molecular fragment data further comprises: determining the first molecular fragment data from the plurality of pieces of molecular fragment data based on at least one of the following:
the degree of shape matching is less than a second threshold; or
the spatial margin is less than a third threshold; or
the polarity data is less than a fourth threshold.

11. The method according to any one of claims 1 to 10, wherein generating the target molecule data representing the target molecule comprises:
generating second molecular fragment data representing a second molecular fragment based on the remaining fragment data and context data that is in the protein data and that is associated with the first molecular fragment data; and
generating the target molecule data based on the second molecular fragment data and the remaining fragment data.

12. The method according to claim 11, wherein generating the second molecular fragment data representing the second molecular fragment comprises:
determining whether the first molecular fragment data is end data representing an end in the three-dimensional structure of the protein;
if the first molecular fragment data is the end data, determining, from the remaining fragment data, first molecular fragment generation information corresponding to the end data; and
generating the second molecular fragment data based on the first molecular fragment generation information and the context data.

13. The method according to claim 11 or 12, wherein generating the second molecular fragment data representing the second molecular fragment comprises:
determining whether the first molecular fragment data is intermediate data representing an intermediate portion of the three-dimensional structure of the protein;
if it is determined that the first molecular fragment data is the intermediate data, determining, from the remaining fragment data, second molecular fragment generation information and third molecular fragment generation information that correspond to the intermediate data; and
generating the second molecular fragment data based on the second molecular fragment generation information, the third molecular fragment generation information, and the context data.

14. The method according to any one of claims 11 to 13, wherein generating the target molecule data comprises:
generating candidate molecule data representing a candidate molecule by adjusting the second molecular fragment data and the remaining fragment data; and
determining the target molecule data from the candidate molecule data based on an attribute of the target molecule.

15. The method according to any one of claims 1 to 14, further comprising:
generating three-dimensional graphic display of the target molecule and the three-dimensional structure of the protein.

16. An apparatus for drug design, wherein the apparatus comprises:
a data obtaining unit, configured to obtain protein data representing a three-dimensional structure of a protein and initial molecule data representing an initial molecule to be bound to the three-dimensional structure of the protein;
a molecular fragment determining unit, configured to determine first molecular fragment data representing a first molecular fragment in the initial molecule based on the protein data and the initial molecule data;
a remaining-fragment determining unit, configured to determine remaining fragment data representing a remaining molecular fragment in the initial molecule other than the first molecular fragment by removing the first molecular fragment from the initial molecule; and
a target-molecule generation unit, configured to generate target molecule data representing a target molecule based on the remaining fragment data and the protein data.

17. A computing device cluster, comprising at least one computing device, wherein each computing device comprises a processor and a memory; and
the processor of the at least one computing device is configured to execute instructions stored in the memory of the at least one computing device, to enable the computing device cluster to perform the method according to any one of claims 1 to 15.

18. A computer-readable storage medium, wherein a computer program is stored on the computer-readable storage medium; and when the program is executed by a processor, the method according to any one of claims 1 to 15 is implemented.

19. A computer program product, comprising computer-executable instructions, wherein when the computer-executable instructions are executed by a processor, the method according to any one of claims 1 to 15 is implemented.
